(19) Europäisches Patentamt
European Patent Office
Office européen des brevets

(11) Publication number: **0 064 821**
**B1**

(12) **EUROPEAN PATENT SPECIFICATION**

(45) Date of publication of patent specification: **09.04.86**

(51) Int. Cl.$^4$: **B 01 J 23/31,** C 07 C 120/14, C 07 C 121/32

(21) Application number: **82301991.4**

(22) Date of filing: **19.04.82**

# (54) Promoted bismuth cerium molybdate catalysts and use thereof in oxidation-type reactions.

(30) Priority: **29.04.81 US 258708**

(43) Date of publication of application:
**17.11.82 Bulletin 82/46**

(45) Publication of the grant of the patent:
**09.04.86 Bulletin 86/15**

(84) Designated Contracting States:
**BE DE FR GB IT NL**

(56) References cited:
**FR-A-2 257 571**
**GB-A-1 160 737**
**GB-A-1 445 512**
**GB-A-2 016 295**
**US-A-3 173 957**
**US-A-3 316 182**
**US-A-4 212 766**

(73) Proprietor: **THE STANDARD OIL COMPANY**
**Midland Building**
**Cleveland, Ohio 44115 (US)**

(72) Inventor: **Brazdil, James Frank**
**5195 Haverford Drive**
**Lyndhurst Ohio 44124 (US)**
Inventor: **Suresh, Dev Dhanaraj**
**1052 Iroquois Run**
**Macedonia Ohio 44056 (US)**
Inventor: **Grasselli, Robert Karl**
**150 Greentree Road**
**Chagrin Falls Ohio 44022 (US)**

(74) Representative: **Smith, Sydney et al**
**Elkington and Fife High Holborn House 52/54**
**High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

The present invention relates to new catalyst systems for ammoxidizing propylene to produce acrylonitrile.

The catalytic ammoxidation of propylene to produce acrylonitrile is a well known process. Many different catalysts have been proposed for this process. While such catalysts are capable of providing excellent yields of acrylonitrile, it is always desirable to provide new catalysts especially effective in this reaction.

In GB—A—1,445,512, there is disclosed a process for the preparation of methacrylonitrile in which isobutene, ammonia and oxygen or a molecular oxygen containing gas are reacted in a gaseous phase in the presence of a catalyst, the catalyst containing the elements in such amounts that its empirical formula is:—

$$Me_vBi_xCe_yMo_{12}O_z$$

in which Me represents one or more of the elements Li, Na, K, Rb, and Cs, v is from 0.1 to 6, x and y are each from 1 to 12, and z has a value taken to satisfy the average valences of the elements in the oxidation states in which they exist in the catalyst.

In FR—A—2,257,571 there is described a catalyst of use in the preparation of acrylonitrile or methacrylonitrile, which catalyst has tellurium as a promoter. This catalyst has the formula:

$$A_xCe_yTe_vMo_{12}O_z$$

in which A represents Fe, Cr, Al, Bi or a mixture of two or more of these metals
x is between 0.3 and 4;
y is between 1.0 and 10; and
v is between 1.0 and 10.

Accordingly, it is an object of the present invention to provide new catalysts which are especially useful in the ammoxidation of propylene to produce acrylonitrile.

We have found that the incorporation of certain elements into known bismuth cerium molybdate redox catalysts remarkably enhances the catalytic properties of such catalysts for use in various oxidation-type reactions such as the ammoxidation of propylene to produce acrylonitrile.

Thus, according to the present invention, known bismuth cerium molybdate redox catalysts are improved by the incorporation therein of a promoting amount of an alkali metal, Tl, Sm, Ag, Cu, Cr, Sb, rare earth, other than Ce and Sm, Te, Ti, Zr, Th or mixture thereof in certain ratios.

The present invention provides a process for ammoxidizing propylene to produce acrylonitrile wherein propylene, ammonia and an oxygen-containing gas are contacted with an oxidation catalyst at elevated temperature to produce said acrylonitrile, characterised in that there is used as catalyst a bismuth cerium molybdate oxide complex oxidation catalyst containing a promoting amount of an alkali metal, Tl, Sm, Ag, Cu, Cr, Sb, rare earth metal other than Ce and Sm, Te, Ti, Zr, Th or mixture thereof, wherein the molar ratios of (Bi+Ce):(W+V+Mo) is in the range from 1/2 to 5/6, inclusive, which catalyst is a single phase material isostructural with lanthanum molybdate.

Catalysts

Bismuth cerium molybdate redox catalysts are known. See, for example, U.S. Patents 3,173,957; 3,262,962 and 3,316,182. In accordance with the invention it has been found that the incorporation of certain additional elements into such catalysts will improve the catalytic properties of these materials. Usually this improvement will be reflected in an increase in product yields, which is a measure of the amount of product produced based on the amount of reactant fed. In some instances, however, this improvement will be reflected in an increase in product selectivity, which is a measure of the ability of the catalyst preferentially to form the desired product as opposed to unwanted byproducts. In any event, incorporation of one or more of these elements, either by adding such elements to a known bismuth cerium molybdate base system or substituting these elements for a part of the bismuth and/or cerium in the known base system, will provide a catalyst with better catalytic properties than the corresponding base system. Furthermore, catalysts of the invention produce byproduct effluents with very low chemical oxidation demand (COD) and are thus environmentally superior to many known redox catalysts such as the iron bismuth molybdates.

The elements found to be effective promoters in accordance with the invention are (I) Sm or a monovalent element such as alkali metals, Tl, Ag and Cu, preferably K, Rb and/or Cs, (2) elements whose most common valence state is 4+, e.g. Te, Ti, Zr and Th, and (3) Cr, Sb and rare earths other than Ce and Sm, such as La, Y, Pr, Nd and Di. These elements can be incorporated into the bismuth cerium molybdate base systems alone or in admixture.

The catalysts of the invention preferably correspond to the following general formula

$$R_rQ_qA_aBi_bCe_cW_dV_eMo_fO_x \qquad (I)$$

wherein

A is alkali metal, Tl, Sm, Ag, Cu or mixtures thereof, preferably K, Rb, Cs or mixtures thereof,

Q is Ti, Zr, Th, Te or mixtures thereof,

R is Cr, Sb or a rare earth element other than Sm and Ce, preferably La, Y, Pr, Nd, Di or mixtures thereof, and

wherein

a is 0 to 6,

b is at least 0,01,

c is at least 0,01,

$0 \leq d+e \leq f$,

d+e+f is 8 to 16,

q is 0 to 24,

r is 0 to 24,

$a+q+r>0$

x is a number sufficient to satisfy the valence requirements of the other elements present, said catalyst being free of the combination of A and Te.

Preferred catalysts are those in which $a+q+r \leq b+c+2$. Also preferred are catalysts which satisfy the relationship $2d+2e+2f=a+3b+3c+4q+3r \pm z$ wherein $z \leq 10$, preferably $z \leq 4$. Of these, those in which $a>0$ are especially preferred.

In this regard, catalysts of special interest are those containing Sm or a monovalent promoter, i.e. an A element and optionally Cr and/or Sb. These catalysts can be defined by the formula:

$$R_rA_aBi_bCe_cW_dV_eMo_fO_x \qquad (2)$$

wherein

A is alkali metal, Tl, Sm, Ag, Cu or mixtures thereof, preferably K, Rb, Cs or mixture thereof; and

R=Cr and/or Sb

wherein

a is greter than zero to 6;

b is at least 0,01;

c is at least 0,01;

r is 0 to 12

$0 \leq d+e \leq f$;

d+e+f=8 to 16; and

x is a number sufficient so that the valence requirements of the other elements present are satisfied.

The catalysts of the invention satisfy the relation

$$\frac{1}{2} \leq \frac{b+c}{f+d+e} \leq \frac{5}{6}$$

Of these catalyst even more preferred are those in which

d+e+f=12 to 12.5;

a is 0.01 to 2;

b is at least 1; and

c is at least 1;

r is 0 to 5.

Still more preferred are those of the above catalysts in which a is 0.02 to less than 0.3, preferably 0.02 to 0.2.

Another interesting group of catalysts containing the monovalent A promoter is those of the above formula (2) which satisfy the relation $2d+2e+2f=a+3b+3c+3r \pm z$, wherein $z \leq 6$, preferably $\leq 2$.

The above formulae in general describe the catalysts produced by the invention. However, as will be appreciated by those skilled in the art, such formulae descriptions do not connote that every material following therein will exhibit superior effectiveness as a catalyst. Rather such descriptions connote only that catalysts of the invention will have compositions corresponding to the above formulas. Those skilled in the art readily understand that a catalyst in order to exhibit good catalytic properties must have an appropriate balance of ingredients and that too much of any one element can drastically reduce its effectiveness or even inactivate the catalyst. The same considerations apply to this invention. The base catalyst systems must be compounded so as to have an appropriate balance of ingredients for catalyzing the specific reaction of interest. In addition, the exact amount of A, R or Q promoter element to be incorporated therein, either by adding the promoter to the existing system or substituting some of the Bi and/or Ce content of the base system with promoter, must also be appropriately selected. In accordance with the invention, the incorporation of the above-described A, R and Q elements into known bismuth cerium molybdate catalysts will exhibit a promoting effect on the catalysts in various oxidation-type

reactions. Those skilled in the art can easily determine how much of a particular A, Q or R elements should be incorporated into a particular bismuth cerium molybdate base system to promote a particular oxidation-type reaction by simple routine experimentation, especially in view of the following working examples.

Also, it has been found that some combinations of A, Q and R elements do not result in catalyst improvements but may indeed drastically reduce catalyst effectiveness. For example, the inclusion of even very small amounts of potassium in a tellurium-containing catalyst will essentially inactivate the catalyst. Obviously, such combinations should also be avoided.

The catalysts of the invention are essentially single-phase systems being isostructural with lanthanum molybdate ($La_2Mo_3O_{12}$). See W. Jeitschko, Acta, Cryst. B29, 2074 (1973). They are therefore different from the known iron bismuth molybdates which are multi-phase materials.

The catalysts of the invention can be used either in unsupported form or supported on suitable carriers such as $SiO_2$, $Al_2O_3$, $BPO_4$, $SbPO_4$, $ZrO_2$, $TiO_2$, Alundum and the like. The catalysts can also be coated on these supports by special techniques known in the art.

These catalysts can be prepared by conventional techniques such as disclosed in U.S. Patent No. 3,642,930. These catalysts are most easily prepared by slurry techniques wherein an aqueous slurry containing all of the elements in the objective catalyst is produced, the water removed from the aqueous slurry to form a precatalyst precipitate or powder and the precatalyst then heated in the presence of an oxygen-containing gas such as air at elevated temperature to calcine the precatalyst thereby forming the catalyst. Liquids other than water, such as $C_1$ to $C_8$ alcohols can also be used to form the precatalyst slurry.

Ammoxidation

The catalysts of the invention find significant use in the ammoxidation of propylene to produce acrylonitrile. This reaction is well known and described, for example, in the above-noted U.S. Patent 3,642,930. In general, the ammoxidation reaction is accomplished by contacting the reactant, oxygen and ammonia with a particular catalyst in the vapour phase. The reaction according to the invention is carried out in the same manner and under the conditions generally set forth in that patent.

In a preferred aspect, the process of the invention comprises contacting a mixture comprising propylene, ammonia and oxygen with the catalyst according to the invention at an elevated temperature and at atmospheric or near atmospheric pressure.

Any source of oxygen may be employed in this process. For economic reasons, however, it is preferred that air be employed as the source of oxygen. From a purely technical view point, relatively pure molecular oxygen will give similar results. The molar ratio of oxygen to the olefin in the feed to the reaction vessel should be in the range of 0.5:1 to 4:1 and a ratio of about 1:1 to 3:1 is preferred.

Low molecular weight saturated hydrocarbons do not appear to influence the reaction to an appreciable degree, and these materials can be present; consequently, the addition of saturated hydrocarbons to the reaction feed is within the scope of this invention. Likewise, diluents such as nitrogen and the oxides of carbon may be present in the reaction mixture without deleterious effect.

The molar ratio of ammonia to olefin in the feed to the reactor may vary between about 0.05:1 to 5:1. There is no real upper limit for the ammonia/olefin ratio, but there is generally no reason to exceed the 5:1 ratio. At ammonia/olefin ratios appreciably less than the stoichiometric ratio of 1:1, various amounts of oxygenated derivatives of the olefin will be formed.

Significant amounts of unsaturated aldehydes, as well as nitriles, will be obtained at ammonia-olefin ratios substantially below 1:1, i.e. in the range of 0.15:1 to 0.75:1. Above the upper limit of this range, the amount of aldehydes produced rapidly decreases. Within the ammonia-olefin range stated, maximum utilization of ammonia is obtained and this is highly desirable. It is generally possible to recycle any unreacted olefin and unconverted ammonia.

Water can also be included in the feed although it is not essential. In some instances, e.g. fixed-bed systems, water may improve the selectivity of the reaction and the yield of nitrile. However, reactions not including water in the feed are within the scope of the present invention and are preferred in the fluid-bed operation.

In general, the molar ratio of added water to olefin, when water is added, is in the neighbourhood of 0.1:1 or higher. Ratios on the order of 1:1 to 6:1 are particularly desirable, but higher ratios may be employed, i.e. up to about 10:1.

The reaction is carried out at an elevated temperature such as 200 to 600°C, preferably 400°C to 550°C, more preferably 420°C to 500°C. The reaction should be carried out at about atmospheric or slightly above atmospheric (2 to 3 atmospheres (2.02 to 3.03 bars)) pressure. In general, high pressure, i.e. above 15 atmospheres (15.19 bars) are not suitable since higher pressures tend to favour the formation of undesirable byproducts.

The apparent contact time is not critical, and contact times in the range from 0.1—40 seconds may be employed. The optimal contact time will, of course, vary depending upon the reactant being used, but in general, contact time of from 1—15 seconds is preferred.

The inventive ammoxidation reaction is carried out in the vapour phase. Normally, the process is conducted on a continuous basis using either a fixed-bed or a fluid-bed catalyst. However, a batch operation can be employed.

The reaction product passing out of the reactor is normally in the form of a vapour. Conventionally, this gaseous reaction product is treated to remove $NH_3$ and then partially condensed either by indirect contact with a cooling medium or direct contact with water to form a liquid phase containing acrylonitrile, acrolein, acrylic acid, HCN and acetonitrile and a vapour phase containing $CO_2$, CO, $N_2$ and $O_2$. The acrylonitrile is then separated from the liquid phase by a number of different techniques such as, for example, distillation or water extraction/distillation. Additional steps can be employed to separately recover HCN and/or acetonitrile from the gross reaction product.

In addition to propylene, other hydrocarbons and oxygenated hydrocarbons can be ammoxidized with the catalysts of the invention. For example, alcohols such as isopropanol, n-propanol, t-butyl alcohol, and aldehydes such as acrolein and methacrolein can be readily converted to nitriles in accordance with the present invention. In addition to propylene, other preferred starting materials are aldehydes and alcohols containing three or four carbon atoms. The general ammoxidation process for converting olefins, alcohols and aldehydes to nitrile is well known and described for example in U.S. 3,456,138.

Process conditions

In carrying out the foregoing processes, any apparatus of the type suitable for carrying out the oxidation reactions in the vapour phase may be employed. The processes may be conducted either continuously or intermittently. The catalyst may be a fixed-bed employing a particulate or pelleted catalyst or, in the alternative, a fluid-bed catalyst may be employed which is normally micro-spheroidal.

Working examples

In order to more thoroughly describe the present invention, the following working examples are presented. In these examples, the following definitions apply:

$$\text{"Yield" means } \frac{\text{moles product formed}}{\text{moles reactant fed}} \times 100$$

$$\text{"Selectivity" means } \frac{\text{moles product formed}}{\text{moles reactant reacted}} \times 100$$

In each of the examples and comparative example, a catalyst having the composition set forth in the following tables was prepared in accordance with a standard laboratory preparation. For example, the catalyst of Examples 1 to 3 was prepared as follows:

42.55 gms. of ammonium heptamolybdate was dissolved in about 100 ml. of distilled water. To this was added 41.59 gms. of a 40% silica sol. In a separate beaker, 38.81 gms. of $Bi(NO_3)_3 5H_2O$, 43.86 gms. of $(NH_4)_4Ce(NO_3)_6$ and 0.20 gms $KNO_3$ were dissolved in 10% aqueous nitric acid. The metal nitrate solution was then slowly added to the ammonium heptamolybdate/silica sol mixture along with approximately 50 ml. of distilled water and the pH of the mixture was adjusted to 3.0 by the addition of concentrated $NH_4OH$. The mixture was then refluxed for 3 hours. After approximately 30 minutes of refluxing, the mixture began to thicken and accordingly an additional 150 ml. of distilled water was added. After refluxing, the mixture was evaporated to dryness on a hotplate, dried at 120°C for 16 hours and then calcined at 290°C for 3 hours and then at 425°C for 3 hours. The partially calcined catalyst was then ground, screened to 25 to 35 mesh and then calcined at 550°C for 16 hours to produce the desired catalyst.

Examples 1 to 10 and Comparative Examples A to C

Eight catalysts according to the invention and three comparative catalysts were tested in the known ammoxidation reaction for producing acrylonitrile from propylene. In each example and comparative example, 5 ml of the catalyst was charged into a 6 ml reactor and contacted with a feed comprising 1 propylene/1.1 $NH_3$/10.6 air/4 $H_2O$ at elevated temperature for a contact time of 3 seconds. The gross reaction product recovered from each experiment was then analyzed.

The composition of the catalysts, the reaction temperatures and the results obtained are set forth in the following Table 1.

TABLE 1
Ammoxidation of propylene

Feed: 1 propylene/1.1 $NH_3$ /10.6 air/4 $H_2O$
Contact time: 3 seconds
Catalyst support: 20% $SiO_2$

| Ex No | Catalyst composition | Temp (°C) | Propy conv | Selec to AN | AN yield (%) | HCN yield (%) |
|---|---|---|---|---|---|---|
| 1 | $K_{0.1}Bi_4Ce_4Mo_{12.05}O_x$ | 430 | 83.6 | 78.4 | 65.5 | 3.1 |
| 2 | " | 445 | 92.8 | 81.5 | 75.6 | 3.3 |
| 3 | " | 460 | 99.3 | 80.4 | 79.8 | 3.1 |
| 4 | $K_{0.05}Cs_{0.02}Bi_4Ce_4Mo_{12.03}O_x$ | 460 | 95.2 | 82.5 | 78.5 | 2.5 |
| 5 | $Tl_{0.03}Bi_4Ce_4Mo_{12}O_x$ | 460 | 98.8 | 80.3 | 79.3 | 3.4 |
| 6 | $Cs_{0.02}Bi_4Ce_4Mo_{12}O_x$ | 445 | 98.4 | 75.6 | 74.4 | 3.6 |
| 7 | $K_{0.1}Bi_9Ce_1Mo_{12}O_x$ | 460 | 95.5 | 83.0 | 79.3 | 2.5 |
| 8 | $K_{0.05}Bi_4Ce_4W_2Mo_{10}O_x$ | 460 | 98.5 | 81.8 | 80.5 | 3.1 |
| A | $Bi_4Ce_4Mo_{12}O_x$ | 430 | 100.0 | 65.8 | 65.8 | 3.0 |
| B | " | 460 | 100.0 | 71.5 | 71.5 | 2.9 |
| C | $Bi_4Ce_4W_2Mo_{10}O_x$ | 430 | 98.6 | 70.5 | 69.5 | 3.5 |
| D | $Bi_9Ce_1Mo_{12}O_x$ | 460 | | 75.7 | 71.9 | 3.1 |
| 9 | $Cs_{0.04}Bi_4Ce_4W_4Mo_8O_x$* | 460 | 99.5 | 82.2 | 81.8 | 3.1 |
| 10 | $Cs_{0.05}Bi_4Ce_4W_2Mo_{12}$* | 460 | 96.5 | 81.6 | 78.8 | 2.8 |
| 11 | $Cs_{0.04}Bi_4Ce_4Sb_1W_2Mo_{10}O_x$* | 460 | 97.9 | 81.7 | 80.0 | 2.0 |

* Catalyst contained 50% $SiO_2$ and was calcined at 650°C.

From the foregoing, it can be seen that the catalysts of the invention provide significant yields of acrylonitrile when used in the conventional ammoxidation reaction. Thus, these catalysts are of significant commercial interest in this field. Moreover, these catalysts are also advantageous because they are redox stable and they provide an environmentally acceptable effluent (i.e. a byproduct effluent with a very low COD).

Comparative Examples E

Example 3 was repeated except that the bismuth content of the catalyst was replaced with tellurium. The results obtained, as well as the results of Example 3 and Comparative Example B, are set forth in the following Table 2.

TABLE 2

| Ex No | Catalyst (+20 $SiO_2$) | Temp (C) | AN yield (%) | Selec to AN |
|---|---|---|---|---|
| B | $Bi_4Ce_4Mo_{12}O_x$ | 460 | 71.5 | 71.5 |
| 3 | $K_{0.1}Bi_4Ce_4Mo_{12}O_x$ | 460 | 79.8 | 80.4 |
| E | $K_{0.1}Te_4Ce_4Mo_{12}O_x$ | 460 | 10.4 | 24.2 |

The above table shows that the addition of alkali metal to the known bismuth cerium molybdate catalyst significantly improves the ability of the catalyst to produce acrylonitrile. In addition, this table further shows that the addition of alkali metal to a corresponding tellurium cerium molybdate catalyst effectively inactivates the catalyst. Specifically, Example 3 and Comparative Example E show that bismuth and tellurium are not equivalent in this system.

Examples 12 to 29 and Comparative Examples F to H

Examples 1 to 10 were repeated using various different R and Q promoters of the invention. In these examples a portion of the cerium and/or bismuth was replaced with the R or Q element so that a stoichiometric balance with respect of molybdenum could be maintained. The results are set forth in the following Table 3.

TABLE 3
Ammoxidation of propylene

Feed: 1 propylene/1.1 $NH_3$/10.6 air/4 $H_2O$
Contact time: 3 seconds
Catalyst support: 20% $SiO_2$

| Ex No | Catalyst composition | Temp (°C) | Propy conv | Selec to AN | AN yield (%) | HCN yield (%) |
|---|---|---|---|---|---|---|
| 12 | $Cr_2Ce_2Bi_4Mo_{12}O_x$ | 430 | 94.4 | 77.2 | 72.9 | 3.4 |
| 13 | " | 445 | 98.6 | 74.9 | 73.8 | 3.6 |
| 14 | $K_{0.05}Cr_2Ce_2Bi_4Mo_{12.03}O_x$ | 460 | 89.5 | 77.6 | 69.4 | 2.6 |
| 15 | $Te_2Ce_2Bi_4Mo_{12}O_x$ | 430 | 98.9 | 72.2 | 71.4 | 3.4 |
| F | $K_{0.05}Te_2Ce_2Bi_4Mo_{12.03}O_x$ | 460 | 68.2 | 50.0 | 34.1 | 0.2 |
| 16 | $La_2Ce_2Bi_4Mo_{12}O_x$ | 430 | 98.7 | 67.3 | 66.4 | 3.1 |
| G | $La_4Bi_4Mo_{12}O_x$ | 430 | 94.6 | 56.0 | 53.0 | 3.7 |
| 17 | $K_{0.05}La_2Ce_2Bi_4Mo_{12.03}O_x$ | 430 | 93.4 | 77.5 | 72.4 | 3.0 |
| 18 | " | 445 | 98.1 | 77.1 | 75.6 | 3.0 |
| 19 | $K_{0.1}La_2Ce_2Bi_4Mo_{12.05}O_x$ | 430 | 75.4 | 83.8 | 63.2 | 3.0 |
| 20 | " | 460 | 90.5 | 81.3 | 73.6 | 2.6 |
| 21 | $K_{0.1}La_4Ce_2Bi_2Mo_{12.05}O_x$ | 445 | 95.7 | 75.3 | 72.1 | 3.0 |
| 22 | " | 460 | 98.8 | 75.1 | 74.2 | 3.0 |
| 23 | $K_{0.1}La_6Ce_1Bi_1Mo_{12.05}O_x$ | 460 | 92.1 | 70.7 | 65.1 | 2.6 |
| 24 | $Y_2Ce_2Bi_4Mo_{12}O_x$ | 420 | 92.4 | 67.3 | 62.2 | 3.9 |
| 25 | $K_{0.05}Y_2Ce_2Bi_4Mo_{12.03}O_x$ | 460 | 98.7 | 76.3 | 75.3 | 2.8 |
| 26 | $K_{0.1}Pr_2Ce_2Bi_4Mo_{12.05}O_x$ | 460 | 94.7 | 76.2 | 72.1 | 6.9 |
| 27 | $K_{0.1}Nd_2Ce_2Bi_4Mo_{12.05}O_x$ | 460 | 97.6 | 75.8 | 74.0 | 2.9 |
| 28 | $K_{0.1}Di_2Ce_2Bi_4Mo_{12.05}O_x$ | 430 | 93.7 | 76.8 | 72.0 | 2.5 |
| 29 | $Cr_2Ce_3Bi_4Mo_{12}O_x$ | 430 | 98.7 | 68.4 | 67.5 | 2.7 |
| H | $Cr_2Ce_3Te_4Mo_{12}O_x$ | 430 | 46.0 | 75.5 | 34.7 | 0.5 |

From the above Table 3 it can be seen that the various R and Q elements set forth above also exert a promoting effect on bismuth cerium molybdate catalysts. In addition, it can be seen that alkali metal, specifically potassium, tends to decrease the activity (conversion) of catalysts but that this activity decline can be largely ameliorated by increasing the temperature without significant loss of acrylonitrile selectivity. However, even at very low concentrations, potassium essentially inactivates a tellurium containing catalyst in that not only is the activity of the catalyst significantly reduced but also its selectivity.

Examples 30, 31 and Comparative Example I

A number of catalysts were used to ammoxidize propylene to produce acrylonitrile and a waste effluent, which was tested to determine chemical oxidation demand. In each example, the gross reactor effluent was scrubbed with aqueous sodium phosphate and the scrubber solution then vacuum distilled to remove acrylonitrile, HCN and acetonitrile. After diluting with water back to original volume, the scrubber solutions were analysed for COD by a technique essentially the same as ASTM D1252. The compositions of the catalyst and the relative COD's obtained are set forth in the following Table 4. In this table, the COD of the prior art iron bismuth molybdate catalyst is taken as 1.00 and the COD's of the other catalysts compared thereto.

TABLE 4
Relative chemical oxygen demand

| Ex No | Catalyst composition | Relative COD |
|---|---|---|
| I | 50% $K_{0.1}Ni_{2.5}Co_{4.5}Fe_3BiP_{0.5}Mo_{12}O_x$+50% $SiO_2$ | 1.00 |
| 30 | 50% $Cs_{0.04}Bi_4Ce_4Mo_8W_4O_x$+50% $SiO_2$ | 0.46 |
| 31 | 80% $K_{0.1}Bi_4Ce_4Mo_{12}O_x$+20% $SiO_2$ | 0.42 |

Table 4 shows that the catalysts of the invention are vastly superior to known complex bismuth molybdate in terms of producing environmentally acceptable effluents. Thus, it will be appreciated that the catalysts of the invention are particularly attractive from a commercial standpoint in that they provide both excellent produce yields (Example 3 and 9 above) and much cleaner waste effluents than known bismuth molybdates.

**Claims**

1. A process for ammoxidizing propylene to produce acrylonitrile wherein propylene, ammonia and an oxygen-containing gas are contacted with an oxidation catalyst at elevated temperature to produce said acrylonitrile, characterised in that there is used as catalyst a bismuth cerium molybdate oxide complex oxidation catalyst containing a promoting amount of an alkali metal, Tl, Sm, Ag, Cu, Cr, Sb, rare earth metal other than Ce and Sm, Te, Ti, Zr, Th or mixture thereof, wherein the molar ratios of (Bi+Ce):(W+V+Mo) is in the range from 1/2 to 5/6, inclusive, which catalyst is a single phase material isostructural with lanthanum molybdate.

2. A process for ammoxidizing propylene to produce acrylonitrile as claimed in claim 1 characterised in that an iron-free oxidation catalyst which has the formula:

$$Bi_bCe_cMo_fO_x$$

wherein
b is at least 0,01;
c is at least 0,01;
f is 8 to 16; and
x is a number sufficient to satisfy the valence requirements of the other elements present, said catalyst further containing a promoting amount of a promoter selected from the group of alkali metal, Tl, Sm, Ag, Cu, Cr, Sb, Te, Ti, Zr, Th or mixtures thereof, said catalyst being free of the combination of Te with alkali metal, Tl, Sm, Ag and/or Cu, is used.

3. A process as claimed in claim 2 characterised in that the promoter is alkali metal, Tl, Sm, Ag, Cu or mixture thereof.

4. A process as claimed in claim 2 characterised in that the catalyst has the formula:

$$R_rQ_qA_aBi_bCe_cW_dV_eMo_fO_x$$

wherein A is alkali metal, Tl, Sm, Ag, Cu or mixtures thereof;
Q is Te, Ti, Zr, Th or mixtures thereof;

R is Cr, Sb or a rare earth IIIB element other than Ce and Sm or mixtures thereof; and wherein

a is 0 to 6;
b is at least 0,01;
c is at least 0,01;
$0 \leq d+e \leq f$;
$d+e+f$ is 8 to 16;
q is 0 to 24;
r is 0 to 24;
$a+q+r>0$
x is a number sufficient to satisfy the valence requirements of the other elements present.

5. A process as claimed in claim 4 characterised in that $2d+2e+2f=a+3b+3c+4q+3r \pm z$ wherein $z \leq 10$.

6. A process as claimed in claim 4 characterised in that the catalyst has the formula:

$$R_rA_aBi_bCe_cW_dV_eMo_fO_x$$

wherein

A is alkali metal;
R is Cr and/or Sb, and

wherein

a is greater than zero to 6;
b is at least 0,01
c is at least 0,01
r is 0 to 12;
$0 \leq d+e \leq f$.

7. A process as claimed in claim 6 characterised in that A is K, Rb, Cs or mixture thereof.

8. A process as claimed in claim 7 characterised in that
$d+e+f=12$ to 12.5;
a is 0.01 to 2;
b is at least 1;
c is at least 1; and
r is 0 to 5.

9. A process as claimed in claim 8 characterised in that a is 0.02 to less than 0.3.

10. A process as claimed in claim 9 characterised in that $2d+2e+2f=a+3b+3c \pm z$, where $z \leq 6$.

11. A process as claimed in claim 6 characterised in that $2d+2e+2f=a+3b+3c \pm z$, wherein $z \leq 6$.

12. A process as claimed in claim 4 characterised in that A is an alkali metal.

13. A process as claimed in claim 12 characterised in that the catalyst is free of Ni, Co and Mg.

14. A process for ammoxidizing propylene to produce acrylonitrile wherein propylene, ammonia and an oxygen-containing gas are contacted with an oxidation catalyst at elevated temperature to produce said acrylonitrile, characterised in that an iron- and tellurium-free oxidation catalyst which has the formula:

$$A_aBi_bCe_cW_dMo_fO_x$$

wherein

A is an alkali metal, Tl, Sm, Ag, Cu or mixture thereof, and

wherein

a is 0.02 to 0.2;
b is 4;
c is 4;
d is 0 to 4;
$d+f$ is 12 to 14; and
x is a number sufficient to satisfy the valence requirements of the other elements present is used.

15. A process as claimed in claim 14 characterised in that A is alkali metal.

16. A process as claimed in claim 15 characterised in that A is potassium and/or cesium.

**Patentansprüche**

1. Verfahren zur Ammoxidierung von Propylen zur Erzeugung von Acrylnitril, worin Propylen, Ammoniak und ein sauerstoffhaltiges Gas mit einem Oxidationskatalysator bei erhöhter Temperatur zur Erzeugung des Acrylnitrils in Berührung gebracht werden, dadurch gekennzeichnet, daß als Katalysator ein Oxidationskatalysator auf Basis eines oxidischen Wismuth-Cer-Molybdatkomplexes verwandt wird, welcher enthält eine aktivierende Menge eines Alkalimetalls, Tl, Sm, Ag, Cu, Cr, Sb, eines anderen Selten-Erd-Metalls als Ce und Sm, Te, Ti, Zr, Th oder einer Mischung derselben, worin die molaren Verhältnisse von (Bi+Ce):(W+V+Mo) im Bereich von einschließlich 1/2 bis 5/6 liegen, wobei der Katalysator ein mit Lanthanmolybdat isostrukturelles Einphasenmaterial ist.

2. Verfahren zur Ammoxidierung von Propylen zur Erzeugung von Acrylnitril nach Anspruch 1, dadurch gekennzeichnet, daß ein eisenfreier Oxidationskatalysator, welcher die Formel aufweist:

$$Bi_bCe_cMo_fO_x$$

worin
b wenigstens 0,01 ist;
c wenigstens 0,01 ist;
f 8 bis 16 ist; und
x eine Zahl ist, die ausreicht, die Valenzbedürfnisse der anderen vorhandenen Elemente zu befriedigen, wobei der Katalysator außerdem eine aktivierende Menge eines Promotors, ausgewählt aus der Gruppe, bestehend aus Alkalimetall, Tl, Sm, Ag, Cu, Cr, Sb, Te, Ti, Zr, Th, oder Mischungen davon, enthält, wobei der Katalysator frei von der Kombination von Te mit Alkalimetall, Tl, Sm, Ag und/oder Cu ist, verwandt wird.

3. Verfahren nach Anspruch 2, dadurch gekennzeichnet, daß der Promotor ein Alkalimetall, Tl, Sm, Ag, Cu oder eine Mischung derselben ist.

4. Verfahren nach Anspruch 2, dadurch gekennzeichnet, daß der Katalysator die Formel aufweist:

$$R_rQ_qA_aBi_bCe_cW_dV_eMo_fO_x$$

worin
A ein Alkalimetall, Tl, Sm, Ag, Cu oder eine Mischung davon ist;
Q Te, Ti, Zr, Th oder eine Mischung davon ist;
R Cr, Sb oder ein anderes Seltenerd-IIB-Element ist als Ce und Sm oder eine Mischung davon ist; und
worin
a 0 bis 6 ist;
b wenigstens 0,01 ist;
c wenigstens 0,01 ist;
$0 \leq d+e \leq f$;
d+e+f 8 bis 16 ist;
q 0 bis 24 ist;
r 0 bis 24 ist;
$a+q+r>0$;
x eine Zahl ist, die ausreicht, die Valenzbedürfnisse der anderen vorhandenen Elemente zu befriedigen.

5. Verfahren nach Anspruch 4, dadurch gekennzeichnet, daß $2d+2e+2f=a+3b+3c+4q+3r\pm z$ ist, worin $z \leq 10$ ist.

6. Verfahren nach Anspruch 4, dadurch gekennzeichnet, daß der Katalysator die Formel aufweist:

$$R_rA_aBi_bCe_cW_dV_eMo_fO_x$$

worin
A ein Alkalimetall ist;
R Cr und/oder Sb ist und
worin
a größer als 0 bis 6 ist;
b wenigstens 0,01 ist;
c wenigstens 0,01 ist;
r 0 bis 12 ist;
$0 \leq d+e \leq f$.

7. Verfahren nach Anspruch 6, dadurch gekennzeichnet, daß A K, Rb, Cs oder eine Mischung davon ist.

8. Verfahren nach Anspruch 7, dadurch gekennzeichnet, daß
$d+e+f=12$ bis 12,5;
a 0,01 bis 2 ist;
b wenigstens 1 ist;
c wenigstens 1 ist; und
r 0 bis 5 ist.

9. Verfahren nach Anspruch 8, dadurch gekennzeichnet, daß a 0,02 bis weniger als 0,3 ist.

10. Verfahren nach Anspruch 9, dadurch gekennzeichnet, daß $2d+2e+2f=a+3b+3c\pm z$ ist, worin $z \leq 6$ ist.

11. Verfahren nach Anspruch 6, dadurch gekennzeichnet, daß $2d+2e+2f=a+3b+3c\pm z$ ist, worin $z \leq 6$ ist.

12. Verfahren nach Anspruch 4, dadurch gekennzeichnet, daß A ein Alkalimetall ist.

13. Verfahren nach Anspruch 12, dadurch gekennzeichnet, daß der Katalysator frei von Ni, Co und Mg ist.

14. Verfahren zur Ammoxidierung von Propylen zur Erzeugung von Acrylnitril, worin Propylen,

Ammoniak und ein sauerstoffhaltiges Gas mit einem Oxidationskatalysator bei erhöhter Temperatur zur Erzeugung des Acrylnitrils in Berührung gebracht werden, dadurch gekennzeichnet, daß ein eisen- und tellurfreier Oxidationskatalysator, der die Formel aufweist:

$$A_aBi_bCe_cW_dMo_fO_x$$

worin

A ein Alkalimetall, Tl, Sm, Ag, Cu oder eine Mischung derselben ist, und

worin

a 0,02 bis 0,2 ist;

b 4 ist;

c 4 ist;

d 0 bis 4 ist;

d+f 12 bis 14 ist; und

x eine Zahl ist, die ausreicht, die Valenzbedürfnisse der anderen vorhandenen Elemente zu befriedigen, verwandt wird.

15. Verfahren nach Anspruch 14, dadurch gekennzeichnet, daß A ein Alkalimetall ist.

16. Verfahren nach Anspruch 15, dadurch gekennzeichnet, daß A Kalium und/oder Cäsium ist.

## Revendications

1. Procédé pour l'ammoxydation du propylène pour produire de l'acrylonitrile, dans lequel du propylène, de l'ammoniac, et un gaz contenant de l'oxygène sont mis en contact avec un catalyseur d'oxydation, à température élevée, pour produire ledit acrylonitrile, caractérisé en ce qu'on utilise comme catalyseur un catalyseur d'oxydation complexe d'oxyde de molybdate de bismuth et de cérium contenant une quantité efficace comme promoteur d'un métal alcalin, de Tl, Sm, Ag, Cu, Cr, Sb, d'un métal des terres rares autre que Ce et Sm, de Te, Ti, Zr, Th ou d'un mélange de ceux-ci, dans lequel les rapports molaires (Bi+Ce):(W+V+Mo) sont dans l'intervalle de 1/2 à 5/6, inclus, lequel catalyseur est une matière à phase unique isostructurale avec le molybdate de lanthane.

2. Procédé pour ammoxyder le propylène pour produire de l'acrylonitrile selon la revendication 1, caractérisé par le fait qu'on utilise un catalyseur d'oxydation exempt de fer qui répond à la formule:

$$Bi_bCe_cMo_fO_x$$

dans laquelle

b est au moins 0,01;

c est au moins 0,01;

f est 8 à 16; et

x est un nombre suffisant pour satisfaire aux exigences de valence des autres éléments présents, ledit catalyseur contenant en outre une quantité efficace comme promoteur d'un promoteur choisi dans le groupe constitué d'un métal alcalin, de Tl, Sm, Ag, Cu, Cr, Sb, Te, Ti, Zr, Th ou de mélanges de ceux-ci, ledit catalyseur étant exempt de la combinaison de Te avec un métal alcalin, Tl, Sm, Ag et/ou Cu.

3. Procédé selon la revendication 2, caractérisé par le fait que le promoteur est un métal alcalin, Tl, Sm, Ag, Cu ou un mélange de ceux-ci.

4. Procédé selon la revendication 2, caractérisé par le fait que le catalyseur répond à la formule:

$$R_rQ_qA_aBi_bCe_cW_dV_eMo_fO_x$$

dans laquelle

A est un métal alcalin, Tl, Sm, Ag, Cu ou des mélanges de ceux-ci;

Q est Te, Ti, Zr, Th ou des mélanges de ceux-ci;

R est Cr, Sb ou un élément des terres rares du groupe IIIB autre que Ce et Sm ou des mélanges de ceux-ci; et

dans laquelle

a est 0 à 6;

b est au moins 0,01;

c est au moins 0,01;

$0 \leq d+e \leq f$

d+e+f est 8 à 16;

q est 0 à 24;

r est 0 à 24;

$a+q+r>0$

x est un nombre suffisant pour satisfaire aux exigences de valence des autres éléments présents.

5. Procédé selon la revendication 4, caractérisé par le fait que $2d+2e+2f=a+3b+3c+4q+3r\pm z$ où $z \leq 10$.

6. Procédé selon la revendication 4, caractérisé par le fait que le catalyseur répond à la formule:

$$R_rA_aBi_bCe_cW_dV_eMo_fO_x$$

dans laquelle

A est un métal alcalin;

R est Cr et/ou Sb, et

dans laquelle

a est plus grand que zéro à 6;

b est au moins 0,01;

c est au moins 0,01;

r est 0 à 12;

$0 \leq d+e \leq f$.

7. Procédé selon la revendication 6, caractérisé par le fait que A est K, Rb, Cs ou un mélange de ceux-ci.

8. Procédé selon la revendication 7, caractérisé par le fait que

d+e+f=12 à 12,5;

a est 0,01 à 2;

b est au moins 1;

c est au moins 1; et

r est 0 à 5.

9. Procédé selon la revendication 8, caractérisé par le fait que a est 0,02 à moins de 0,3.

10. Procédé selon la revendication 9, caractérisé par le fait que $2d+2e+2f=a+3b+3c\pm z$ ou $z \leq 6$.

11. Procédé selon la revendication 6, caractérisé par le fait que $2d+2e+2f=a+3b+3c\pm z$, ou $z \leq 6$.

12. Procédé selon la revendication 4, caractérisé par le fait que A est un métal alcalin.

13. Procédé selon la revendication 12, caractérisé par le fait que le catalyseur est exempt de Ni, Co et Mg.

14. Procédé pour ammoxyder le propylène pour produire de l'acrylonitrile, dans lequel du propylène, de l'ammoniac et un gaz contenant de l'oxygène sont mis en contact avec un catalyseur d'oxydation, à une température élevée, pour produire ledit acrylonitrile, caractérisé par le fait qu'on utilise un catalyseur d'oxydation exempt de fer et de tellure répondant à la formule

$$A_aBi_bCe_cW_dMo_fO_x$$

dans laquelle

A est un métal alcalin, Tl, Sm, Ag, Cu ou un mélange de ceux-ci, et

dans laquelle

a est 0,02 à 0,2;

b est 4;

c est 4;

d est 0 à 4;

d+f est 12 à 14; et

x est un nombre suffisant pour satisfaire aux exigences de valence des autres éléments présents.

15. Procédé selon la revendication 14, caractérisé par le fait que A est un métal alcalin.

16. Procédé selon la revendication 15, caractérisé par le fait que A est du potassium et/ou du césium.